# EUROPEAN PATENT SPECIFICATION

(11) **EP 3 377 621 B1**
(45) Date of publication and mention of the grant of the patent: **25.09.2019**
(21) Application number: 16798137.2
(22) Date of filing: 16.11.2016
(51) Int. Cl.: A41D 19/00, C12N 1/20, C12N 11/02

(54) **A WEARABLE ARTICLE AND A METHOD FOR PRODUCING A WEARABLE ARTICLE**
TRAGBARER GEGENSTAND UND VERFAHREN ZUR HERSTELLUNG EINES TRAGBAREN GEGENSTANDES
ARTICLE PORTABLE ET PROCÉDÉ POUR LA PRODUCTION D'UN ARTICLE PORTABLE

(30) Priority: 16.11.2015 GB 201520195
(43) Date of publication of application: 26.09.2018
(73) Proprietor: Century International Enterprises Limited, Hong Kong (CN); Cornelissen, Ruddi, 3930 Hamont (BE); Maas, Bernard, 3930 Hamont (BE)
(72) Inventor: CORNELISSEN, Ruddi, 3930 Hamont (BE); MAAS, Bernard, 3930 Hamont (BE)
(74) Representative: Beck Greener LLP
(86) International application number: PCT/EP2016/077892
(87) International publication number: WO 2017/085139

(56) References cited:
- WO-A1-2004/037305
- US-B1- 6 264 967
- BEESEMANS K ET AL: "P14.19 Contamination of non-sterile gloves by Bacillus cereus and non-spore forming bacteria", JOURNAL OF HOSPITAL INFECTION, ELSEVIER, AMSTERDAM, NL, vol. 76, 10 October 2010 (2010-10-10), pages S50-S51, XP027367175, ISSN: 0195-6701 [retrieved on 2010-10-01]

## Description

The present invention relates to a wearable article and to a method for producing a wearable article. Document WO 2004/037305 discloses a glove according to the preamble of claim 1.

In order to be allowed to provide their services, healthcare practices must comply with national hygiene standards and be subject to regulation by government organisations such as the Care Quality Commission in England. Over the past several years a number of studies, focussed both in the UK and overseas, have put a spotlight on the prevalence of healthcare-associated infections (or HAIs) in surgeries and hospitals. The term HAI encompasses any infection contracted within a healthcare setting or brought from elsewhere into such a setting. Some of the most common causes of HAIs include MRSA, MSSA, *C.diff* and *E.coli* and norovirus. As a result of a greater public awareness of the problem, new measures have been introduced in order to reduce the spread of these infections. These measures have worked their way into the existing protocol, with which all healthcare practices are bound to comply. Measures introduced in the UK over the past several decades have meant that protocol relating to hygiene, in particular to hand hygiene, has tightened considerably.

The World Health Organisation, for example, published the final version of their guidelines on hand hygiene in health care in 2009 and the Health Act 2006 introduced a code of practice aimed at preventing and controlling HAIs. Hand hygiene is now heavily regulated and healthcare professionals are required to clean their hands prior to and following contact with each patient. Workers must also wear gloves in situations where they are likely to come into contact with blood or other bodily fluids. In such situations gloves are required to be changed between patients and, in some circumstances, after touching potentially contaminated objects and during the treatment of a single patient. Hands must be washed prior to donning gloves and again after the gloves have been removed.

Although adhering to proper hygiene practices in order to stem the spread of HAIs is seen as vital, regular immersion in water and repeated washing of hands with irritants such as soaps, alcohols, and detergents can lead to the onset of certain debilitating skin conditions. One of the most common conditions widespread among healthcare professionals is irritant contact dermatitis (ICD). This inflammatory skin condition, caused by exposure to detergents, results in red and itchy skin in the initial stages and, if not treated, can worsen and lead to weeping and cracking. Cracks and open wounds in the skin greatly increase the chances of infection by bacteria that are more often present in hospitals and other similar environments.

Several studies, such as that published by Stocks et al. in the British Journal of Dermatology (2015; 173, pp165-171), attribute the increase in reported cases of ICD among healthcare workers in recent years to the stricter protocols regarding hand hygiene and the introduction of campaigns such as the cleanyourhands campaign in the UK. Similar campaigns and regulations have been introduced elsewhere so that the prevalence of ICD among healthcare workers is an issue worldwide and not just in the UK.

In addition to this, it is common for workers to suffer from allergies which are related to regular contact with the materials from which items of protective clothing are formed. This is the case particularly with regard to medical grade gloves which are traditionally made from latex. Gloves produced using other materials, such as nitrile and vinyl, are becoming more widely available however vinyl tends to be leakier than latex and nitrile is still a more expensive alternative. Proper donning of the gloves is important to prevent ripping and contamination. In order to make gloves easier to apply and to keep hands dry, powders such as corn starch have been added to the interior. Corn starch, however, can adhere to latex proteins and become airborne, causing asthma and other more serious allergic reactions in users. Lubricants such as polymer coatings are also commonly used to smooth the inside of medical gloves, as described in WO-A-2005/110,749.

Employers in healthcare establishments are responsible for the well-being of their employees and as such are obliged to take measures to combat the onset of dermatitis, allergic reactions and other skin conditions arising as a consequence of having to adhere to a strict hygiene protocol.

Neutral PH soaps can be less irritating to skin than alkaline alternatives and have been introduced in some hospitals. As an alternative to washing with detergents or soap, alcohol gels and foam based sanitizers can be rubbed onto hands in order to disinfect them. These gels tend to irritate skin to a lesser extent but are not suitable in certain situations (such as when the spread of norovirus is a risk). Persons at risk are often advised to apply moisturising or emollient cream to hands to reduce drying of the skin resulting from dermatitis. Practitioners, however, will often forget to apply emollient if it is simply provided in dispensers, and its application does not form part of current hospital protocol.

The glove described in US-A-2015/089,714 has a moisturising coating on the skin contacting surface. The moisturising agent may be aloe vera and serves to moisturise and soothe affected skin during wear.

US-A-2007/104,766 describes an elastomeric article such as a glove for use by healthcare professionals. This glove includes a water based coating into which an antimicrobial agent is introduced. The antimicrobial agent can act to reduce the levels of bacteria (of all kinds) on the treated surface. GB2501940 describes a similar article.

The term skin flora refers to the microorganisms generally present on the skin, largely in the superficial layers and hair follicles. This surface layer of skin flora will be mainly non-pathogenic (and the levels of pathogenic to non-pathogenic bacteria tend to remain fairly constant in a normal person). Regular cleaning of the skin with detergents, intended to reduce the levels of pathogenic bacteria on the skin surface, will also reduce levels of harmless or food-grade (GRAS) bacteria forming part of a person's normal skin flora. Without competition for nutrients from other types of bacteria, transient pathogenic bacteria will tend to thrive much more easily. Repeated washing of hands with detergents, applying and removing gloves and rewashing will tend to disturb the natural balance of the skin flora, increasing the likelihood of infection and of damage to the skin.

In an article by Vandini et al. (2014) entitled "Hard Surface Biocontrol in Hospitals Using Microbial-Based Cleaning Products" the authors set out the results of a study into the effectiveness of applying non-pathogenic microorganisms to cleaning products for use on hard surfaces in hospitals. Spores of *Bacillus subtilis, Bacillus pumilus* and *Bacillus megaterium* were introduced into cleaning products and used in three hospitals over the course of several months and on a range of hard surfaces. In comparison with surfaces cleaned using traditional detergent based products, the surfaces cleaned using the microorganism containing substances were found to harbour much lower levels of many common pathogenic bacteria (a 60-70% reduction) as well as decreased levels of resistance to antibiotics in the microorganisms tested.

According to a first aspect of the present invention, there is provided a glove formed of an elastomeric material and having a surface, wherein a stabilized probiotic is provided on a part of the surface to form an active layer, and the part of the surface contacts the skin of the wearer.

The invention provides an elastomeric glove that can replenish or supplement the levels of beneficial bacteria on the skin during normal wear. These can compete with pathogenic microbes to help to control levels of harmful bacteria. When the wearable article is a glove of the kind that protocol demands must be worn by medical personnel, the user, simply by following protocol, will help to recolonise their skin with probiotics such as those that are naturally present on the skin's surface. An active layer is provided which can restore the microbiological equilibrium of the skin's surface. The glove therefore provides a particularly effective way to prevent the onset of dermatitis and similar conditions and prevents affected skin from becoming infected more easily. The inactive glove becomes an active, preventive article.

The user does not have to remember to carry out additional procedures. They also do not need to touch anything other than the glove after washing their hands, which precludes any risk of contact with further harmful substances. Accordingly, the wearer is simply required to follow current hospital hygiene protocol without the need to take any additional steps which might result in practitioners neglecting to carry out the required actions.

By reducing levels of pathogenic material on the skin surface the risks of infection are also reduced. This is particularly important where skin may be affected by irritant contact dermatitis due to repeated washing with detergents. Furthermore, the likelihood of transferring pathogenic microorganisms to a patient or another surface after removal of the item is reduced.

Protective clothing such as medical grade gloves must be formed of a material that will prevent pathogens from touching the skin of the wearer so that the article can act as a barrier to cross-infection. Elastomeric materials such as latex, vinyl or nitrile are particularly suitable. Applying the active layer of the invention to elastomeric wearable articles of the type commonly used in hospitals will also require a minimum or even no changes to be made to the production process, thus ultimately reducing the cost of the article.

Elastomeric gloves are among the most frequently used disposable item of protective clothing in healthcare environments. As explained above, current protocol relating to hand hygiene can cause various disorders of the skin. (Stocks et Al. (British Journal of Dermatology (2015, 173 pp 165-171)). Providing the active probiotic layer on gloves can help to combat these disorders, prevent infection, and restore microbiological equilibrium of the hand.

Applying the active layer on a skin contacting surface allows the probiotics to be transferred onto the skin of the wearer, increasing the levels of probiotics on their skin, thus also increasing the amount of beneficial bacteria.

In an embodiment, the active layer is provided on the whole of the skin contacting surface. From a manufacturing point of view this is simple, and applying the active layer to the whole of the skin contacting surface (such as the whole of the interior surface of a glove) increases the area of the skin onto which probiotics are transferred, maximising the beneficial effects for the wearer.

In an embodiment, the active layer is applied so as to be substantially uniform. Again, a uniform layer is easier to achieve and can simplify production and automation of the production process.

In an embodiment, the active layer extends to a depth of 0.01 to 10 microns below the surface. Embedding vesicles within the surface (or partially embedding them) can improve sticking to the surface, however at least some vesicles must be near enough to the surface to be able to transfer probiotics onto the skin once burst. If the active layer is applied to the article prior to hardening during production, the active layer may extend some way into the surface of the article. In an embodiment, the active layer has a thickness in the range of 1 micron to 0.5 mm.

In an embodiment, the probiotics can comprise microbes that make up part of a normal person's skin flora. This way the skin flora's natural balance or equilibrium can be restored. Many harmless bacteria forming part of the normal skin flora, such as *staphylococcus epidermis,* can compete effectively with pathogenic bacteria to help to keep their levels to a minimum. *Staphylococcus epidermis* is particularly effective in reducing levels of *staphylococcus aureus,* which can be pathogenic. It is not imperative to use probiotics that generally form part of the skin flora. Any harmless or food-grade bacteria can be used. Certain bacteria have been found to help reduce inflammation and these may also be beneficial when used as part of the active layer.

The concentration of probiotics in the active layer may be similar to the concentration of bacteria on the skin of a normal person's hand or may be designed so as to transfer this amount to the skin. In an embodiment, the concentration of probiotics in the active layer is in the range 500-5000 CFU per cm². This allows enough of the viable microorganisms to be transferred to the skin of the wearer to restore levels of harmless bacteria to a level where they are able to compete with pathogenic microorganisms. Ideally, the overall number and concentration of probiotics in the active layer will be as high as possible to ensure that pathogenic bacteria can be kept to a minimum level. The concentration applied to the article will of course also depend on cost and practicality of manufacturing and storing the article.

In an embodiment, the probiotics can be encapsulated within vesicles. This may help to aid stabilisation and means that when the article is worn, vesicles can burst or dissolve in order to transfer probiotics onto the skin. The vesicles also help to hold the probiotics on the surface of the glove during transport and storage.

In an embodiment, the vesicles have an average largest measurement across of between 1 and 300 microns. The plurality of vesicles is in the form of a powder, fluid, cream, or gel which is easy to apply to the article during manufacture.

In an embodiment, at least some of the vesicles burst when the article is worn to transfer at least some of the probiotics to the skin of the wearer. The probiotics are reactivated once on the skin and are able to reproduce.

In an embodiment, the active layer is in the form of a powder. In an embodiment, the active layer is in the form of a fluid, cream or gel. The powder can also be added to a fluid, cream or gel prior to application onto the surface.

In an embodiment, the active layer has been applied to the article when the surface is at a temperature of between 27 and 60 degrees centigrade. The elastomeric article will not be completely hardened at this temperature, which means that the probiotic containing layer can adhere to the surface (or in some cases be partially embedded within the surface). Once hardened, the active layer is held on the surface with the probiotics in a stabilized state until the wearer puts on the article.

In embodiments, the active layer has been sprayed onto the surface using one or more spray guns. Spraying provides an even layer of the probiotic mixture. By controlling the time over which spraying occurs, the thickness of the layer can also be easily managed.

Ultrasonic techniques can also be used to put the active layer onto the surface. The advantage using the ultrasonic technique is that the surface of the glove in contact with the skin of the wearer does not feel wet, and is more comfortable.

In embodiments, the active layer further comprises an adhesive substance. This adhesive can assist bonding of the vesicles or stabilized probiotics to the surface and help to prevent the active layer from being rubbed off before use.

According to a second aspect of the present invention, there is provided a wearable article having a surface, wherein a stabilized probiotic is provided on a part of the surface.

According to a third aspect of the present invention, there is provided a method for producing an elastomeric glove, the method comprising: forming the material for making the glove into a desired shape; and providing stabilized probiotic material on a skin contacting surface of the glove to form an active layer.

In embodiment, providing the material on at least part of the surface comprises spraying the material onto the surface using a spray gun.

In an embodiment, the material is provided on the surface when the surface is at a temperature of between 27 and 60 degrees centigrade.

In an embodiment, the method further comprises, prior to providing the material on the surface, encapsulating the probiotic material within vesicles.

In an embodiment, the method further comprises allowing the glove to cool and harden.

In an embodiment, a former is used to form the desired shape and the method further comprises peeling the glove from the former so that the exterior surface when on the former becomes the interior surface of the glove. In the case of an elastomeric glove, the existing method of manufacture is substantially unchanged. Only the addition of a step in which the active layer is provided is required. This way existing machinery and techniques can still be used.

In an embodiment, the stabilized probiotic material is formed into a mist using ultrasonic atomization before being applied to the glove. This allows an extremely even coating of the probiotic containing solution to be applied.

In an embodiment, the mist is formed within a tunnel and the gloves are passed through the tunnel to apply the probiotic substance to the surface.

In an embodiment, the gloves are passed through the tunnel on a conveyor belt.

According to a fourth aspect of the present invention, there is provided a method for producing a wearable article, the method comprising: forming the material for making the wearable article into a desired shape; and providing stabilized probiotic material on at least part of a surface of the article.

Embodiments of the present invention will now be described in detail with reference to the accompanying drawings, in which:
Figure 1 shows a cross section through the surface of a glove;
Figure 2 illustrates one possible size distribution of vesicles in the active layer;
Figure 3A shows a cross section through the surface of a wearable article, wherein vesicles are embedded in the surface;
Figure 3B shows a cross section through the surface of a wearable article, wherein adhesive is applied to the surface before applying the probiotic substance;
Figure 3C shows a cross section through the surface of a wearable article, wherein vesicles are not embedded within the surface and wherein the thickness of the active layer varies;
Figure 4 shows a glove with an active layer on the skin contacting surface as the glove is being peeled from a former;
Figure 5 shows vesicles on the surface of a wearable article bursting due to stretching of the surface;
Figure 6 shows a method of coating a plurality of articles with probiotics during manufacture;
Figure 7A shows a prior art elastomeric glove;
Figure 7B shows an elastomeric glove with a probiotic layer on the inner surface;
Figure 8A shows the normal situation of the human hand skin prior to disturbance where there is a healthy balance between beneficial and possibly pathogenic bacteria;
Figure 8B shows the disturbed situation of the human hand skin where there is a disturbed balance between beneficial and possible pathogenic bacteria;
Figure 8C shows the human hand skin after application of the glove with probiotic layer: restored microbiological balance leading to a normal healthy situation.

Gloves formed from elastomeric materials are used in healthcare practices and when carrying out veterinary work in order to prevent the spread of pathogens and to protect patients and workers from infection. As mentioned above, current protocol dictates that these gloves must be worn whilst carrying out certain procedures and must be changed after each procedure or if contact is made with substances that are likely to be infectious. The same is also true of most other personal protective equipment worn in such environments such as facemasks, eye protection and any other item of protective clothing. Although the specific examples in the description below refer to a glove or gloves, the invention is applicable to any other article, which may be a protective article, wherein probiotics are applied to any skin contacting surface. Here a skin contacting surface refers to any part of the article that is skin facing and could touch the skin when the article is worn. Some parts of the skin contacting surface may not always actually touch the skin. As an example, in the case of a glove the entire interior surface of the glove would be referred to as skin contacting. However, when worn by some users the interior surface at the very tips of the fingers or in a crease may not actually ever directly contact the wearer.

An active layer of stabilized probiotics can also be applicable to garments worn outside of the healthcare environment. The sole of a gym shoe or an incontinence garment, for example, could comprise an active layer including probiotics which could help to reduce the growth of bad-smelling or harmful bacteria. An efficient way to apply the active layer to a gym shoe might be to apply the probiotics in a spray form. A spray bottle or a bottle with holes in the lid containing the stabilized probiotics in liquid or powder form could be sold to the user so that the layer could be reapplied easily.

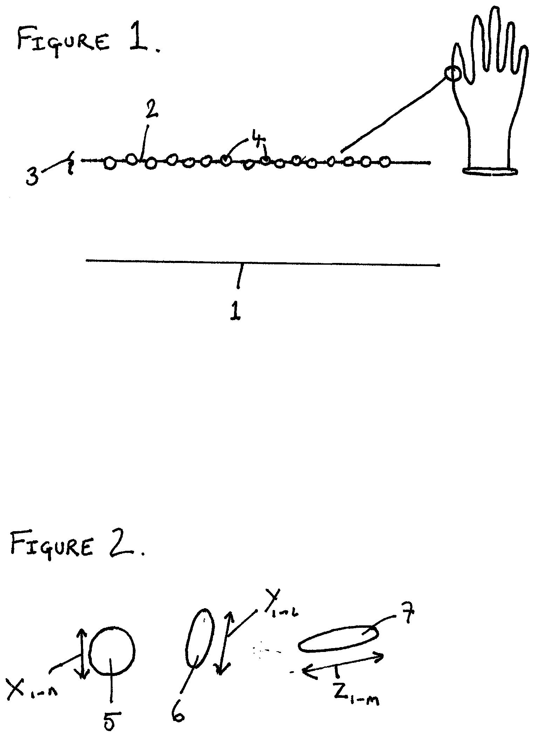

Figure 1 shows the cross-section of a part of the surface of a glove having an active probiotic layer. Active in this context refers to the fact that at least some of the microbes within the layer are viable. It is desirable for all the microbes in the layer are viable just after the production process is complete. Preferably, at least 90% of the microbes in the layer are viable at this point, and more preferably at least 98%. The cross section is taken through the whole surface from the exterior 1 to the interior 2. It is the interior surface 2 which will contact the hand of the wearer. In the figure, a layer of probiotic material 3 is applied to the interior surface 2 of the glove only. Here the probiotic material is held within substantially spherical vesicles 4 of diameter between 1 and 300 microns. The thickness of the layer 3 may be slightly larger than the diameter of the vesicles since the latter may not all penetrate the surface 2 to the same extent. In embodiments, the thickness of layer 3 can be varied by applying more vesicles to a particular part of the surface. Vesicles may be provided with (or formed from) a substance that allows them to attach to one another to hold the layer to the glove's surface. The layer 3 can also be thicker in some parts of the surface than others.

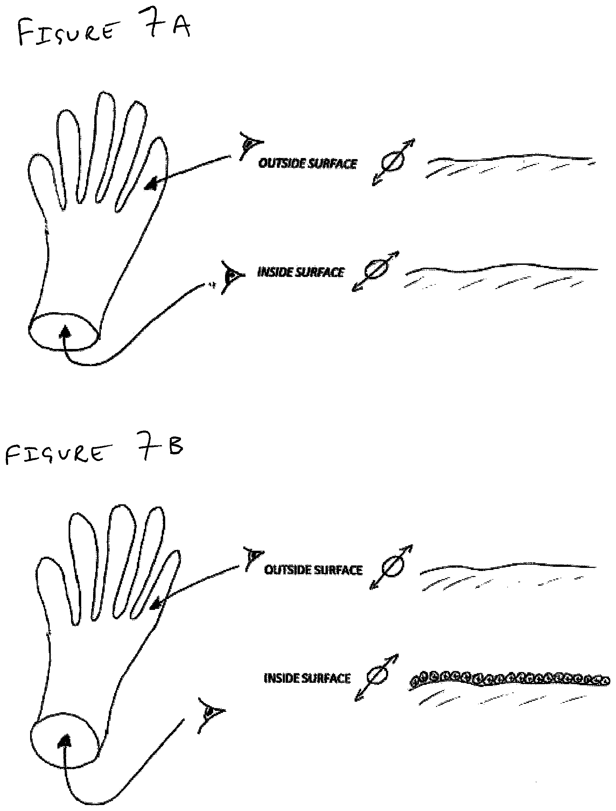

Figure 7A illustrates a prior art elastomeric glove with a cross section of both the inner and outer surfaces of the glove. Neither the inside nor the outside surface of the glove includes a layer of probiotics, thus only the elastomeric material from which the glove is formed will contact the skin of the wearer during use. Figure 7B shows an elastomeric glove according to embodiments of the invention. As described above a layer of probiotics is provided on a surface of the glove. In this case the layer is provided on the inner surface in order that the probiotics contact, and can be transferred to, the skin of the wearer.

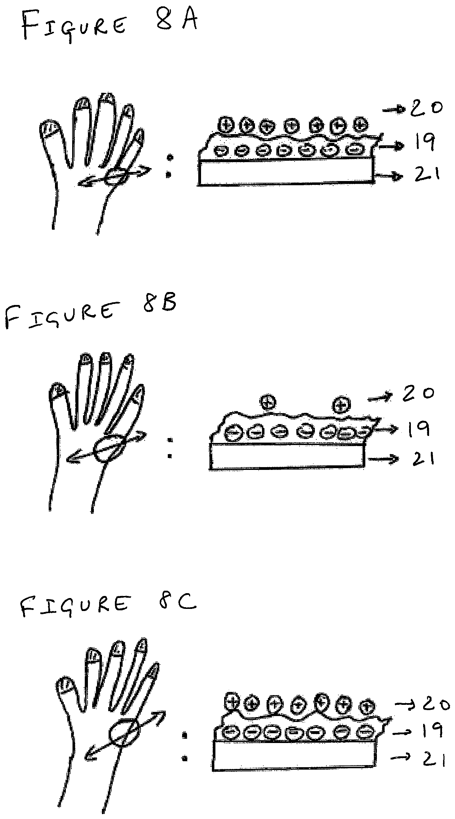

Figures 8A to 8C illustrate the effect on a wearer's skin flora of a glove according to the present invention. Figure 8A illustrates a typical, healthy situation of the skin with normal, balanced levels flora or microbiota. The surface of the skin 21 comprises a biofilm or protective layer 19 within which anaerobic bacteria may be protected. Beneficial bacteria (indicated with a plus sign) on the surface layer 20 are susceptible to disturbances such as washing of the hands and can be removed or destroyed easily by this type of activity. Figure 8B illustrates the effect of such a disturbance on the skin flora of the hand. While bacteria protected by the biofilm are unaffected, much of the surface flora has been removed or may have been killed off if a bactericide has been applied to the skin during cleaning. In general, beneficial bacteria will be affected to a greater extent by typical disturbances to the skin so that the relative amounts of beneficial to harmful bacteria will be decreased. The lack of beneficial bacteria as a result of disturbance thus leaves the skin vulnerable and may result in infection. Figure 8C shows a cross-section through the skin of a hand after a glove with a probiotic layer on the inner, skin contacting, surface has been worn by the user. The skin flora has been returned to the situation prior to disturbance and a healthy level of beneficial bacteria on the skin surface has been restored.

Probiotics can be applied only to the interior surface of the glove, only to the exterior of the glove or to both the interior and the exterior of the glove. It is preferable that at least the interior surface is provided with an active layer because it is the practitioner who is likely to be suffering from conditions such as irritant contact dermatitis which will make them more susceptible to infection and transfer of the microorganisms to the skin will be more effective for the wearer. Applying probiotics to the outside of medical gloves may be problematic in that conditions on the skin or body of a patient must be carefully controlled and applying additional substances may interfere with this environment.

The normal skin flora contains both resident and transient microorganisms and in a normal person there may be 1000s of bacteria (or 1000s of colony forming units) present on one square centimetre of skin. Of the resident bacteria, *staphylococcus epidermis* is the most common with corynebacteria such as diphtheroids also playing a prominent role, particularly on the palm of the hand. Some of the bacteria forming part of the normal skin flora, such as *staphylococcus aureus,* can be pathogenic while others are considered harmless. The amount of transient bacteria on an individual's skin can vary greatly from person to person and will depend on the level of moisture and acidity of the skin as well as the temperature. The proportion of different microbes present does, though, tend to be fairly consistent for each adult. As well as variations between populations on the skin of different individuals, the prevalence of different microbes depends on the part of the body inhabited. Normal flora can be divided broadly into three types in each of which the natural levels of different types of bacteria is balanced differently. The hands, face, and trunk fall into the same category which means that the combinations, proportions and concentrations of bacteria which make up normal skin flora for the hands will also represent something close to the normal skin flora on the face and trunk.

The different probiotics within the active layer applied to the wearable article are preferably harmless as well as being able to compete as effectively as possible with pathogenic bacteria on the skin. The active layer applied when forming an elastomeric glove may be manufactured to represent as closely as possible the normal proportion of each type of bacteria (or each type of non-pathogenic bacteria) found on the skin of the hand. The levels could be different for an article designed to contact a different part of the body. There is obviously a fair amount of variation in the proportions and quantities of the different types of bacteria present in the skin flora of different people and so an average should be taken. "Normal" skin flora here thus refers to the average amount of each microbe that would be observed in a spread of people covering all sectors of the world's population. There may, in any case, be a significant spread in the values that can be considered "normal".

Alternatively, only the most prominent or the most prominent few bacteria commonly present on the skin may be used in the active layer in order to simplify production. The normal skin flora is highly dependent on the region of the body concerned and, as mentioned above, the face, trunk and hands tend to display a similar balance of at least the most prevalent bacteria. This means that applying the same composition to an active layer within articles intended to contact the face, hands and trunk can still represent (at least to an extent) the natural skin flora of these regions without having to alter the make-up of the active layer. Probiotics can also be chosen to represent those that will most effectively help to control pathogenic growth on the skin. It is not necessary that these form a part or a substantial part of normal skin flora, for example *Bacillus subtilis, Bacillus pumilus* and/or *Bacillus megaterium* can be used, as for, and possibly in similar proportions to those used in the cleaning products mentioned above.

Because the "normal" skin flora may also vary in its make up depending on certain characteristics of the wearer, in an embodiment articles may be specifically designed to replicate the composition of the skin flora expected for that particular type of person. On the other hand, since use of different compositions for the active layer may be costly and time consuming, it will likely be more efficient to provide a single composition for use in all articles produced.

The concentration as well as the relative proportion of different probiotics present in the active layer may be made to represent the normal skin flora (or to supplement this) and as bacteria may be present in amounts of over 1000 colony forming units per square centimetre on the skin (depending which part of the body is concerned). A higher concentration, for example a concentration of around 5000 colony forming units per square centimetre, may also be applied to the surface of the wearable article to allow for death of some organisms during manufacture, storage, and transport and for a failure to transfer to the skin of the wearer (due to containing vesicles remaining intact, for example).

The probiotics applied to the surface of a wearable article must be capable of surviving throughout the period during which the product is stored and distributed. Medical gloves and other disposable supplies are generally purchased in bulk and stored until they are required. Depending on the number of staff and the rate at which gloves are used the gloves may need to be stored for months or even years. The material must therefore be stabilized to allow them to survive for long periods.

Stabilizing refers to limiting the exposure of microorganisms to stimulating environmental conditions such as warmth and moisture in order that their numbers remain fairly constant and they can remain viable. Methods of stabilizing probiotics have been studied widely. This has generally been with a view to their use in the pharmaceutical industry as ingestible substances for rebalancing gut flora. Probiotics have traditionally required storage at below room temperature. While it is possible to store medical gloves and the like in a cooled environment such as a fridge, this is costly and not ideal. More recent methods of stabilization do, however, allow probiotics to survive at room temperature for extended periods. WO-A-2005/047489, for example, describes a method for producing a fine powder containing probiotics which can remain stabilized at room temperature. Freeze or spray dried bacteria concentrate is added to a stabilizer having a low water content in order to achieve this.

In a preferred embodiment the probiotic material is in an encapsulated form when applied to the glove or wearable article. The probiotic substance is thus contained in a plurality of microscopic vesicles which can help to protect the probiotics from the environment. These vesicles must be strong enough to survive transport and storage but must be able to release probiotics when the glove or article is worn to release these on to the surface of the skin.

One commonly used method for producing encapsulated probiotics (usually for use in edible capsules) is by spray-drying. Probiotics in suspension within a matrix are atomized and then dried by directing the atomized particles at high velocities through a stream of hot air. Protectants can be added to reduce the chances of probiotics dying due to the heat of the drying step, however these will not usually be completely effective and the success of this method will depend on the particular cocktail of probiotics used. Spray-cooling uses similar techniques, but employs a cold rather than a hot stream of air to dry the particles.

Fluid-bed agglomeration and coating involves fluidising particles of the material to be coated and then spraying with an atomized substance. Coating materials are generally lipid based but carbohydrates and proteins can also be used. Freeze drying is another widely used encapsulation technique. During freeze drying, particles containing the probiotics along with a carrier material are subjected to freezing temperatures followed by sublimation of water from the particle in a vacuum. A cryoprotectant (or cryoprotectants) can be added to prevent the cold temperatures from killing the microbes. Vacuum drying works similarly but without reducing the temperature to below zero.

During emulsification, water is mixed well with a hydrophobic substance such as oil (along with an emulsifier) and probiotics are trapped within droplets of the oil. Other known techniques used to produce encapsulated particles include coacervation, extrusion, and dripping, among others. These are described in detail in, for example, the article "Encapsulation Technology to Protect Probiotic Bacteria" by Chavarri, Marañón and Villaran.

All of the above techniques can result in a stabilised mixture and may be suitable for use in producing a wearable article having an active probiotic layer; however any method for stabilising probiotics can be used.

Vesicles that are substantially spherical will generally result from some of the techniques described above. Spherical vesicles for use in the present invention will preferably have diameters in the range of 1-300 microns. If the vesicles used are not spherical, or a mixture of shapes is included, then the average size of the largest measurement across for all of the particles may be in the range of 1-300 microns. If the vesicles have an elongated form, for example, the particles may measure 1-300 microns on average along their longest dimension. Figure 2 shows a mixture made up of vesicles having three different shapes 5, 6, and 7 which have three different largest measurements across (x, y and z respectively). There are n particles with shape 5, I particles with shape 6 and m particles with shape 7. The average largest measurement across can be calculated as (X₁ +...Xₙ) + (Y₁ +...Yₗ) + (Z₁ + ...Zₘ)/n+l+m. This average should be in the range of 1-300 microns. Of course, there may be many more different shapes within the mixture than the three shown in the figure.

Although a stabilised probiotic substance which is not contained within vesicles can be used to produce the wearable article of the invention, vesicles are easy to apply, as will be described in more detail below, and will maintain the probiotic material in a stable state on the surface of the glove or article until pressure is applied by the skin of a wearer. In order to apply the stabilized probiotics to the surface of an elastomeric article a powder, fluid, cream or gel comprising the vesicles must be applied to the surface at the correct stage during manufacture. It is vital that the temperature of the article and environment during application is not so high that the probiotics within the vesicles are killed. More specifically, it is preferable that at least 90% of the microbes are still viable immediately following manufacture of the article. In embodiments, in order to bind the vesicles to the article, it is also preferable that the material of the article is not completely cured or solidified at the time of application. In this way an active layer of probiotic material is applied to the surface and may be embedded to some extent within it. The active layer can also include additives such as hydrating substances or adhesives.

The glove or other wearable elastomeric article may be formed of a polymeric/elastomeric material such as latex, nitrile, vinyl, neoprene, polyurethane, butyl, any other elastomeric material or a combination of two or more materials, the combination of which has elastomeric properties. These articles are generally manufactured using dip moulding techniques. In the case of latex or nitrile gloves, for example, the raw material is a water-based emulsion of rubber particles to which sulphur is added which helps to initiate cross-linking and cause polymers to form. Formers (or moulds) having the desired shape are dipped into the modified emulsion and the emulsion is allowed to dry. The thickness of the skin formed on the former will depend on the viscosity of the emulsion into which the former is dipped, the speed of dipping and/or the number of times the former is dipped. After cooling, the article is removed from the former to finish. Processes for forming articles from other elastomeric materials are similar. These are well known in the art and are not described herein. It is worth mentioning, however, that the production of the elastomeric article will generally involve a cooling or hardening step.

The probiotic vesicles should be applied during the normal manufacture of an elastomeric article at a point when the cooling or hardening process is not yet complete and thus the material of the article is not fully hardened. At this point the vesicles may embed themselves to an extent into the surface of the article as shown in Figure 1 or may stick to the slightly tacky surface. Vesicles can be applied during the cooling process so that they will not be exposed to further heating after application. The optimum temperature range for the material forming the article at the point of application is between 27 and 60 degrees centigrade. This temperature may vary slightly depending on the type of material used. The temperature should be high enough that the material is not completely solidified but low enough that a large proportion (above 98%) of the microbes present can remain viable. Since the probiotics are protected to some extent by the surface of the vesicle and it will take some time for all of the material within the coating to be brought up to the temperature of the surface, the optimum temperature of application can be higher than the temperature at which each individual microbe would be expected not to survive. This higher temperature allows a better adhesion of the vesicles to the surface.

Preferably, the vesicles will be sprayed onto the article at the optimum temperature or optimum hardness of the surface using a nozzle having small holes through which vesicles can pass. Preferably the nozzle holes will be sized so that the probiotic mixture can be easily applied while still being separated sufficiently to provide an even coating. If a uniform coating over the whole surface of the article is desired, the spray gun can be rotated around the article while it is on the former (or the article and former rotated in combination) in order to cover the whole surface. If only portions of the surface are desired to be coated then a template can be held over the article in order to spray only the required regions.

Generally, a substantially uniform coating covering the whole of the article will be the most effective and the most efficient in that it will result in the least amount of wastage. Spraying smaller regions, such as only the interior parts of a glove that will contact fingertips and palms, will save on material whilst still protecting from colonisation by pathogens after glove removal (these being the regions most likely to come into contact with pathogenic material). Several spray guns orientated around the article can also be used to avoid the need for rotation. In order to achieve an even coating the spray gun nozzles will preferably be located at a distance of between 5 and 200 centimetres from the surface of the article at the point at which the article is closest to the spray gun nozzle concerned, however this will depend on the type of spray gun used. Using spray guns in this way allows for easy application of the probiotic layer with minimal adaption of the traditional manufacturing processes used to produce the wearable elastomeric articles that are currently available. The spraying process can be completely automated to allow it to be integrated into an existing production line.

It is also possible to add an adhesive material to the plurality of vesicles in order to better adhere the probiotic layer to the article. The mixture with adhesive can be applied before the surface material has cooled completely (for example with a spray gun as described above). Alternatively, the mixture with adhesive can be applied after curing and cooling of the article. Adhesive can be applied to the surface of the elastomeric article post-cure and the vesicles applied on top of this. Vesicles applied after the article has cured and cooled will not be embedded within the surface. This may make them more likely to burst during storage and their attachment to the surface may not be as strong.

Vesicles can be provided in a fluid, gel or cream (which may be produced in certain embodiments by adding vesicles in the form of a powder to the fluid, gel or cream) and these can also be applied to the surface of the article either during or after curing. The use of a fluid, gel or cream may be particularly advantageous where the article to which the probiotic layer is applied is formed of latex as it may prevent aerosols from being released when the wearer removes the article. However, applying the vesicles before cooling in powder form will also achieve this affect because they will be held in place on the surface. Probiotics in a fluid solution can be sprayed onto the surface of the article as described above.

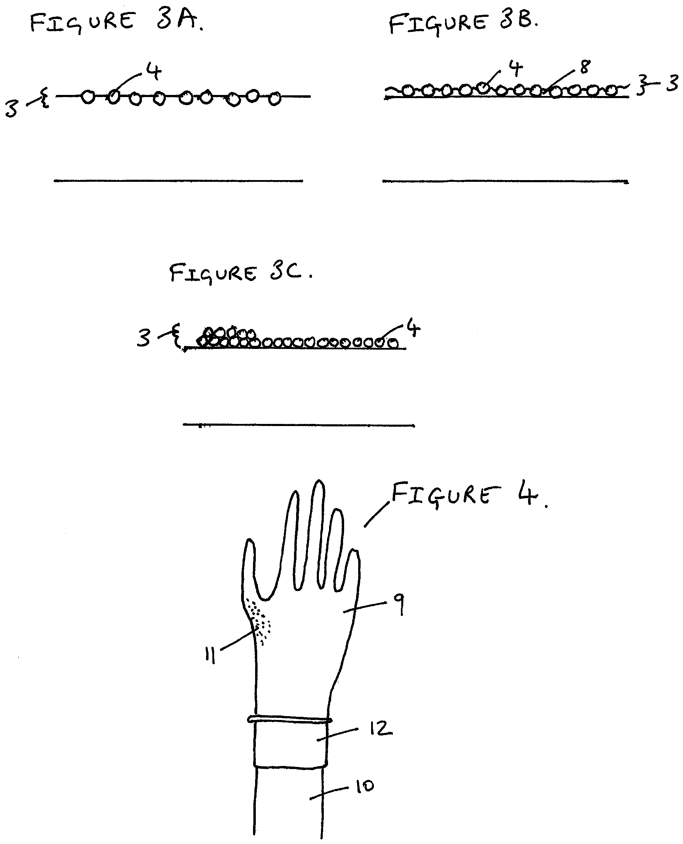

Whichever way the probiotic material is applied, the elastomeric article resulting from the processes described above will have an active probiotic layer provided on at least a part of the surface. In this context the active layer refers to a region either above the surface of the elastomeric article or partly extending into the surface of the elastomeric article (depending on the application technique used) and including the probiotic material. Figures 3A and 3B show the surface of a glove in cross section. In Figure 3A the probiotic material (here shown in vesicles 4) is applied prior to curing the surface material or when the material is slightly soft. In 3B an adhesive substance 8 is applied to the surface of the cured surface probiotic prior to application of the vesicles. The vesicles may extend slightly outward of the adhesive so that once the adhesive has hardened, the vesicles can still burst to release their contents. The active layer in figure 3A extends into the surface, 2, itself and in 3B it does not. Here the active layer 3 is shown having a thickness similar or slightly larger than the diameter of the vesicles; however the active layer can be thicker. In figure 3C the vesicles have been adhered to the surface simply by sticking to the slightly molten material of the article prior to hardening. This can be achieved without the addition of an adhesive even if the material is not soft enough for vesicles to embed themselves. Vesicles may stick to each other to an extent to allow the formation of a thicker layer (adhesive can be added to help with this if the material forming the shell of the vesicles is not sufficiently resinous). The active layer in figure 3C, for example, comprises a thicker and a thinner portion.

During manufacture of an elastomeric glove the former is in the shape of a hand and removal of the article from the former will be by peeling, so that the outer surface of the article when on the former will end up as the interior surface of the glove. The process of peeling the glove 9 from the former 10 is shown in Figure 4. Probiotic containing vesicles 11 are visible on the outer surface of the glove at this point and the interior surface 12 where the glove is being peeled back will ultimately become the exterior. This means that treating the interior surface of the final article can be achieved easily during manufacture by treating the exterior surface prior to removal from the former. If it is desired to treat the exterior surface of a glove then the glove can be manufactured as explained above, removed from the former by peeling and then turned inside out again to return the treated surface to the exterior of the glove (either on or off the former). In order to apply vesicles to both the inner and the outer surface, the material may need to be reheated to the optimum temperature for application or (provided that the temperature of the elastomeric material during manufacture will not be too high) the probiotic material can be applied to the former.

The vesicles may comprise an outer coating or skin to contain the probiotics. This coating must be strong enough to allow the vesicle to hold its form during storage but weak enough that the vesicle can burst when the article contacts the skin of the wearer. In embodiments, the skin may be formed of a material that is soluble or part-soluble so that moisture on the users hand will dissolve the vesicle coating to release the probiotics held inside. The resulting articles in this case will need to be stored in very dry conditions which may not always be possible.

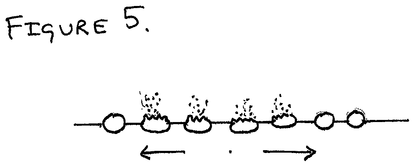

Deformation of the containing elastomeric material which occurs when the article is worn can also provide a force on the vesicles which can burst them. In general, the article will be small enough that it will need to be stretched slightly to fit over the relevant body part (such as the hand in the case of an elastomeric glove). Because vesicles are embedded into the surface material and will tend to attach themselves to it once the material has cured, the stretching of the material surrounding the vesicles may be enough burst them once they are worn as shown in Figure 5. Here the surface material 2 of the glove is being stretched in the direction indicated by the arrows to burst vesicles 4 and release the probiotics or probiotic containing mixture 13.

In embodiments, the vesicles are embedded deep enough in the surface of the article that pressure applied perpendicular to the surface will not be sufficient to burst the vesicles. This way articles can be stored so that they are laid one on top of another without risk of vesicles bursting and the probiotics becoming destabilized. In this case stretching of the material will be required to break the vesicles when the wearer puts on the wearable article. In other embodiments the vesicles can burst on application of pressure perpendicular to the surface. If this is the case then the article does not need to stretch to such an extent when worn (vesicles are broken under pressure from the skin of the wearer, in particular during use). If there is likely to be enough perpendicular force on the vesicles during storage then the articles may need to be kept in boxes or in smaller piles in order that pressure is not applied to burst the vehicles during transport or storage.

A preferred method for providing the probiotic layer on elastomeric gloves is described below. Although the specific example of an elastomeric glove is described, the method can be used to apply a probiotic layer to other articles.

The method comprises passing gloves on their formers through a tunnel containing a thin mist made up of droplets of a fluid containing the probiotics. This mist is produced using an ultrasonic atomization technique. Ultrasonic nozzles make use of high frequency vibrations (produced by piezoelectric transducers) to set up waves in a film at the nozzle tip. Once a particular amplitude for the waves is reached, droplets of the film fall off forming a mist. This technique allows an extremely fine mist to be produced and characteristics of the mist (such as particle size) can be extremely accurately controlled by adjusting the properties of the vibrations used.

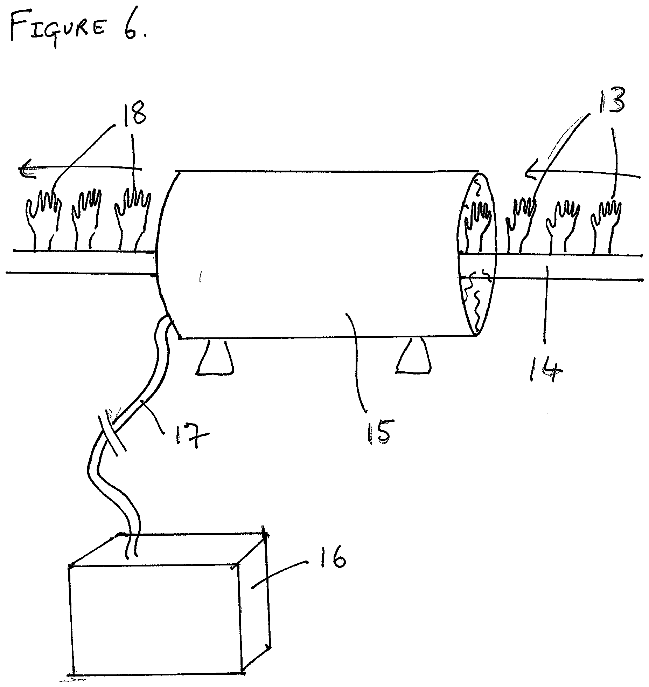

Figure 6 illustrates this method. The figure shows a production line wherein a plurality of gloves 13, still on the formers, are passed along a conveyor belt 14 into a tunnel 15. Reservoir 16 holds the probiotic containing fluid which flows or is pumped along a vessel or tube 17 to spray nozzles within the tunnel. The probiotic fluid is applied to the gloves within the tunnel using the ultrasonic technique described above to produce a mist or fog of small droplets of the fluid that is contained within the tunnel. As the gloves and formers pass through the tunnel, droplets attach to the surface of the gloves forming a probiotic layer thereon. Treated gloves 18 exit the tunnel.

The tunnel may be formed of any waterproof material and may be permanently erected or collapsible and removable to provide some flexibility. In addition, the tunnel need not necessarily be cylindrical and can be any shape, for example a cuboid. In place of a conveyor belt, gloves may be hung from a moving frame and passed through the tunnel in this way.

Although the application method will work with one spraying apparatus only, it is preferable for several apparatus (each with one or more spray nozzles) to be spaced around and along the inner surface of the tunnel for application of an even coat of fluid to the gloves. Nozzles may be positioned around the entrance to the tunnel (and/or around the exit) and may be positioned at equal intervals around the mouth of the tunnel. For example, twelve spraying apparatus may be positioned at 30 degree intervals around a circular entrance and/or exit. In addition, spray apparatus may be positioned at intervals along the inside of the tube, for example every meter. Several tubes may pass from the reservoir to different nozzles or systems of nozzles positioned at intervals along the tunnel or the tube may comprise several outlets leading to each of the nozzles. Preferably, nozzles are spaced at equal intervals along the tunnel and around the circumference or perimeter to provide as even a coating as possible.

Because the density of the mist contained within the tunnel is fairly consistent, the resulting coating of probiotic fluid applied will uniform or close to uniform. The outer surface of the glove while on the former will eventually form the inner surface of the glove (which will contact the skin of the wearer during use) so that the coating applied within the tunnel is to the eventual inner surface of the glove. The thickness of the coating can be adjusted as desired by changing the length of the tunnel or the density of the mist produced within it.

In addition, the length of the tunnel may need to be adjusted depending on the speed of the production line (which may affect the speed at which the conveyor belt moves) to ensure that a coating of the desired thickness is applied. The possibility of adjusting the length of the tunnel to suit the production process ensures that the manufacturing process is changed as little as possible by application of the probiotic layer. Incorporating this step into a traditional manufacturing process should thus be cheap and straightforward, such that the benefits of providing a glove having the probiotic layer will outweigh any additional cost in producing, and therefore in obtaining the gloves.

The gloves can be passed through the tunnel during the normal hardening process. As a result the application of the probiotic layer does not increase the manufacturing time and requires minimal adjustment to the process currently used to produce traditional elastomeric gloves without a probiotic layer. While the probiotics can be applied by submerging gloves in a bath of probiotic fluid or using a conventional spray pistol, the ultrasonic spraying technique described above is preferable in that it does not slow down the production process and it provides a dry feel to the final product which is more comfortable for the wearer.

Embodiments of the present invention have been described with particular reference to the examples illustrated. However, it will be appreciated that variations and modifications may be made to the examples described within the scope of the present invention as defined by the claims.

## Claims

1. A glove (9) formed of an elastomeric material and having a surface (2), **characterized in that** a stabilized probiotic is provided on a part of the surface (2) to form an active layer (3), and the part of the surface contacts the skin of the wearer when the glove is worn.

2. A glove (9) according to claim 1, wherein the active layer (3) is provided on the whole of the skin contacting surface.

3. A glove (9) according to any of claims 1 and 2, wherein the active layer (3) is applied so as to be substantially uniform.

4. A glove (9) according to any of claims 1 to 3, wherein the active layer (3) extends to a depth of 0.01 to 10 microns below the surface.

5. A glove (9) according to any of claims 1 to 4, wherein the active layer (3) has a thickness in the range 1 micron to 0.5mm.

6. A glove (9) according to any of claims 1 to 5, wherein the probiotics comprise microbes that make up part of a normal person's skin flora.

7. A glove (9) according to any of claims 1 to 6, wherein the concentration of probiotics in the active layer (3) is in the range 500 to 5000 CFUs per cm².

8. A method for producing an elastomeric glove (9), the method comprising:
forming the material for making the glove into a desired shape; **characterized by**
providing stabilized probiotic material on a skin contacting surface (2) of the glove to form an active layer (3).

9. A method according to claim 8, wherein providing the material on at least part of the surface (2) comprises spraying the material onto the surface using a spray gun.

10. A method according to any of claims 8 and 9, wherein the material is provided on the surface (2) when the surface is at a temperature of between 27 and 60 degrees centigrade.

11. A method according to claim 10, further comprising allowing the glove (9) to cool and harden.

12. The method of any of claims 8 to 11, wherein a former (10) is used to form the desired shape and the method further comprises peeling the glove (9) from the former (10) so that the exterior surface when on the former becomes the interior surface of the glove.

13. The method of any of claims 8 to 12, wherein the stabilized probiotic material is formed into a mist using ultrasonic atomization before being applied to the glove (9).

14. The method of claim 13, wherein the mist is formed within a tunnel (15) and the gloves are passed through the tunnel to apply the probiotic substance to the surface.

15. The method of claim 14, wherein the gloves are passed through the tunnel (15) on a conveyor belt (14).

## Patentansprüche

1. Handschuh (9), aus einem Elastomermaterial gebildet und eine Oberfläche (2) aufweisend, **dadurch gekennzeichnet, dass** ein stabilisiertes Probiotikum auf einem Teil der Oberfläche (2) bereitgestellt wird, um eine aktive Schicht (3) zu bilden, und der Teil der Oberfläche die Haut des Trägers beim Tragen des Handschuhs berührt.

2. Handschuh (9) nach Anspruch 1, wobei die aktive Schicht (3) auf der gesamten Hautkontaktfläche bereitgestellt wird.

3. Handschuh (9) nach einem der Ansprüche 1 und 2, wobei die aktive Schicht (3) aufgetragen wird, um im Wesentlichen einheitlich zu sein.

4. Handschuh (9) nach einem der Ansprüche 1 bis 3, wobei sich die aktive Schicht (3) bis auf eine Tiefe von 0,01 bis 10 Mikrometer unterhalb der Oberfläche erstreckt.

5. Handschuh (9) nach einem der Ansprüche 1 bis 4, wobei die aktive Schicht (3) eine Dicke in dem Bereich von 1 Mikrometer bis 0,5 mm aufweist.

6. Handschuh (9) nach einem der Ansprüche 1 bis 5, wobei die Probiotika Mikroben umfassen, die Teil der normalen Hautflora einer Person sind.

7. Handschuh (9) nach einem der Ansprüche 1 bis 6, wobei die Konzentration der Probiotika in der aktiven Schicht (3) in dem Bereich von 500 bis 5000 CFU pro cm² liegt.

8. Verfahren zum Herstellen eines Elastomerhandschuhs (9), wobei das Verfahren den folgenden Schritt umfasst:
Bilden des Materials, um den Handschuh in eine gewünschte Form zu bringen;
**gekennzeichnet durch** das Bereitstellen eines stabilisierten probiotischen Materials auf einer Hautkontaktfläche (2) des Handschuhs, um eine aktive Schicht (3) zu bilden.

9. Verfahren nach Anspruch 8, wobei das Bereitstellen des Materials auf mindestens einem Teil der Oberfläche (2) das Sprühen des Materials auf die Oberfläche unter Verwendung einer Sprühpistole umfasst.

10. Verfahren nach einem der Ansprüche 8 und 9, wobei das Material auf der Oberfläche (2) bereitgestellt wird, wenn sich die Oberfläche auf einer Temperatur zwischen 27 und 60 Grad Celsius befindet.

11. Verfahren nach Anspruch 10, ferner umfassend das Kühlen- und Aushärtenlassen des Handschuhs (9).

12. Verfahren nach einem der Ansprüche 8 bis 11, wobei ein Formgeber (10) verwendet wird, um die gewünschte Form zu bilden, und das Verfahren ferner das Abziehen des Handschuhs (9) von dem Formgeber (10) umfasst, so dass die äußere Oberfläche, wenn sie sich auf dem Formgeber befindet, zur inneren Oberfläche des Handschuhs wird.

13. Verfahren nach einem der Ansprüche 8 bis 12, wobei das stabilisierte probiotische Material unter Verwendung von Ultraschallzerstäubung zu einem Nebel gebildet wird, bevor es auf den Handschuh (9) aufgetragen wird.

14. Verfahren nach Anspruch 13, wobei der Nebel innerhalb eines Tunnels (15) gebildet wird und die Handschuhe durch den Tunnel gegeben werden, um die probiotische Substanz auf die Oberfläche aufzutragen.

15. Verfahren nach Anspruch 14, wobei die Handschuhe auf einem Förderband (14) durch den Tunnel (15) gegeben werden.

## Revendications

1. Gant (9) formé d'un matériau élastomère et ayant une surface (2), **caractérisé par le fait qu'**un probiotique stabilisé est disposé sur une partie de la surface (2) pour former une couche active (3), et la partie de la surface entre en contact avec la peau du porteur lorsque le gant est porté.

2. Gant (9) selon la revendication 1, dans lequel la couche active (3) est disposée sur l'ensemble de la surface de contact avec la peau.

3. Gant (9) selon l'une quelconque des revendications 1 et 2, dans lequel la couche active (3) est appliquée de façon à être sensiblement uniforme.

4. Gant (9) selon l'une quelconque des revendications 1 à 3, dans lequel la couche active (3) s'étend à une profondeur de 0,01 à 10 microns sous la surface.

5. Gant (9) selon l'une quelconque des revendications 1 à 4, dans lequel la couche active (3) a une épaisseur comprise dans la plage allant de 1 micron à 0,5 mm.

6. Gant (9) selon l'une quelconque des revendications 1 à 5, dans lequel les probiotiques comprennent des microbes qui constituent une partie de la flore cutanée d'une personne normale.

7. Gant (9) selon l'une quelconque des revendications 1 à 6, dans lequel la concentration de probiotiques dans la couche active (3) est comprise dans la plage allant de 500 à 5000 CFU par cm².

8. Procédé de production d'un gant élastomère (9), le procédé comprenant :
former le matériau pour constituer le gant en une forme souhaitée ;
**caractérisé par** la disposition d'un matériau probiotique stabilisé sur une surface de contact de peau (2) du gant pour former une couche active (3).

9. Procédé selon la revendication 8, dans lequel la disposition du matériau sur au moins une partie de la surface (2) comprend pulvériser le matériau sur la surface à l'aide d'un pistolet pulvérisateur.

10. Procédé selon l'une quelconque des revendications 8 et 9, dans lequel le matériau est disposé sur la surface (2) lorsque la surface est à une température comprise entre 27 et 60 degrés centigrade.

11. Procédé selon la revendication 10, comprenant en outre autoriser le gant (9) à refroidir et durcir.

12. Procédé selon l'une quelconque des revendications 8 à 11, dans lequel une forme (10) est utilisée pour obtenir la forme souhaitée et le procédé comprend en outre enlevage par pelage le gant (9) à partir de la forme (10) de telle sorte que la surface extérieure, lorsque sur la forme, devient la surface inférieure du gant.

13. Procédé selon l'une quelconque des revendications 8 à 12, dans lequel le matériau probiotique stabilisé est formé en un brouillard à l'aide d'une atomisation ultrasonore avant d'être appliqué sur le gant (9) .

14. Procédé selon la revendication 13, dans lequel le brouillard est formé à l'intérieur d'un tunnel (15) et les gants sont amenés à passer à travers le tunnel pour appliquer la substance probiotique à la surface.

15. Procédé selon la revendication 14, dans lequel les gants sont amenés à passer à travers le tunnel (15) sur un transporteur à courroie (14).
